# FASCICULE DE BREVET EUROPEEN

(11) **EP 2 292 739 B1**
(45) Date de publication et mention de la délivrance du brevet: **12.09.2012**
(21) Numéro de dépôt: 10170872.5
(22) Date de dépôt: 19.03.2007
(51) Int. Cl.: C12N 15/113, C12N 15/85, C12N 7/00

(54) **Procédé de préparation de cellules aviaires differenciées et gènes impliqués dans le maintien de la pluripotence**
Verfahrung zur Herstellung differenzierter Zellen vom Huhn, und Gene, die im Erhalt der Pluripotenz impliziert sind
Method for preparing differentiated avian cells and genes involved in the maintenance of pluripotency

(30) Priorité: 24.03.2006 FR 0602597
(43) Date de publication de la demande: 09.03.2011
(62) Demande divisionnaire de: 07727050.2
(73) Titulaire: INSTITUT NATIONAL DE LA RECHERCHE AGRONOMIQUE, 75007 Paris (FR); Centre National de la Recherche Scientifique (CNRS), 75016 Paris (FR); ENS - Ecole Normale Supérieure de Lyon, 69007 Lyon (FR)
(72) Inventeur: Pain, Bertrand, 63830 NOHANENT (FR); Lavial, Fabrice, 69003 LYON (FR); Samarut, Jacques, 69003 LYON (FR)
(74) Mandataire: Tetaz, Franck Claude Edouard

(56) Documents cités:
- PETITTE J N ET AL: "AVIAN PLURIPOTENT STEM CELLS", MECHANISMS OF DEVELOPMENT, ELSEVIER SCIENCE IRELAND LTD, IE, vol. 121, no. 9, 1 septembre 2004 (2004-09-01), pages 1159-1168, XP009077049, ISSN: 0925-4773, DOI: DOI:10.1016/J.MOD.2004.05.003
- PAIN B ET AL: "Long-term in vitro culture and characterisation of avian embryonic stem cells with multiple morphogenetic potentialities", DEVELOPMENT, COMPANY OF BIOLOGISTS, CAMBRIDGE,, GB, vol. 122, no. 8, 1996, pages 2339-2348, XP002164729, ISSN: 0950-1991
- SATO FUMINORI ET AL: "Application of RNA interference to chicken embryos using small interfering RNA", JOURNAL OF EXPERIMENTAL ZOOLOGY, vol. 301A, no. 10, 1 octobre 2004 (2004-10-01), pages 820-827, XP002402499, ISSN: 0022-104X
- HYSLOP L ET AL: "Downregulation of NANOG induces differentiation of human embryonic stem cells to extraembryonic lineages", STEM CELLS, ALPHAMED PRESS, DAYTON, OH, US, vol. 23, no. 8, 1 septembre 2005 (2005-09-01), pages 1035-1043, XP008102678, ISSN: 1066-5099, DOI: DOI:10.1634/STEMCELLS.2005-0080 [extrait le 2005-06-27]
- NAKAMURA HARUKAZU ET AL: "Gain- and loss-of-function in chick embryos by electroporation", MECHANISMS OF DEVELOPMENT, vol. 121, no. 9, septembre 2004 (2004-09), pages 1137-1143, XP002617433, ISSN: 0925-4773
- CANON S ET AL: "Germ cell restricted expression of chick nanog", DEVELOPMENTAL DYNAMICS, WILEY-LISS, INC., NEW YORK, NY, US, vol. 235, no. 10, 1 octobre 2006 (2006-10-01), pages 2889-2894, XP002606925, ISSN: 1058-8388, DOI: DOI:10.1002/DVDY.20927 [extrait le 2006-08-18]
- LAVIAL FABRICE ET AL: "The Oct4 homologue PouV and Nanog regulate pluripotency in chicken embryonic stem cells", DEVELOPMENT (CAMBRIDGE), vol. 134, no. 19, octobre 2007 (2007-10), pages 3549-3563, XP002617434,

### SEQUENCE LISTING

<110> INRA, CNRS, ENS Lyon, Vivalis
<120> Procède de préparation de cellules aviaires différenciées et genes impliques dans le maintien de la pluripotence
<130> D24075-350998
<150> FR 06/02597
   <151> 2006-03-24
<160> 10
<170> PatentIn version 3.3
<210> 1
   <211> 295
   <212> PRT
   <213> Gallus gallus
<400> 1
   Met His Val Lys Ala Lys Asn Leu Leu Arg Met Cys Lys Trp Leu Lys
   1               5                   10                  15
   Gly Leu Arg Asn Ala Arg Gly Ser Thr Trp Gly Arg Ser Gly Gly Arg
               20                  25                  30
   Lys Pro Met Arg Ser Ser Gly Arg Leu Pro Arg Ser Ala Asp Pro Gly
           35                  40                  45
   Trp Gly Asn His Ala Asn Arg Ala Ala Val Val Thr Arg Gly Ile Ser
       50                  55                  60
   Ser His Ser Pro Arg Val Cys Leu Cys Leu Cys Gln Asp Ala Pro Thr
   65                  70                  75                  80
   Ser Glu Glu Leu Glu Gln Phe Ala Lys Asp Leu Lys His Lys Arg Ile
                   85                  90                  95
   Met Leu Gly Phe Thr Gln Ala Asp Val Gly Leu Ala Leu Gly Thr Leu
               100                 105                 110
   Tyr Gly Lys Met Phe Ser Gln Thr Thr Ile Cys Arg Phe Glu Ala Leu
           115                 120                 125
   Gln Leu Ser Phe Lys Asn Met Cys Lys Leu Lys Pro Leu Leu Gln Arg
       130                 135                 140
   Trp Leu Asn Glu Ala Glu Asn Thr Asp Asn Met Gln Glu Met Cys Asn
   145                 150                 155                 160
   Ala Glu Gln Val Leu Ala Gln Ala Arg Lys Arg Lys Arg Arg Thr Ser
                   165                 170                 175
   Ile Glu Thr Asn Val Lys Gly Thr Leu Glu Ser Phe Phe Arg Lys Cys
               180                 185                 190
   Val Lys Pro Ser Pro Gln Glu Ile Ser Gln Ile Ala Glu Asp Leu Asn
           195                 200                 205
   Leu Asp Lys Asp Val Val Arg Val Trp Phe Cys Asn Arg Arg Gln Lys
       210                 215                 220
   Gly Lys Arg Leu Leu Leu Pro Phe Gly Asn Glu Ser Glu Gly Val Met
   225                 230                 235                 240
   Tyr Asp Met Asn Gln Ser Leu Val Pro Pro Gly Leu Pro Ile Pro Val
                   245                 250                 255
   Thr Ser Gln Gly Tyr Ser Leu Ala Pro Ser Pro Pro Val Tyr Met Pro
               260                 265                 270
   Pro Phe His Lys Ala Glu Met Phe Pro Pro Pro Leu Gln Pro Gly Ile
           275                 280                 285
   Ser Met Asn Asn Ser Ser His
       290                 295
<210> 2
   <211> 888
   <212> DNA
   <213> Gallus gallus
<400> 2
   atgcatgtaa aagccaaaaa cctgctgcga atgtgtaaat ggcttaaagg attgcgaaat      60
   gcccgcggca gcacttgggg ccgtagcggt ggcaggaagc cgatgcgctc gagtgggcgg     120
   ctgccccgaa gtgcggatcc tggctggggg aaccacgcga accgcgcggc tgttgtcacc     180
   cgcggtatct cgagccattc accgcgtgtc tgcctctgcc tctgccagga tgcgccaacc     240
   tcagaggagc tggagcagtt tgccaaggac ctcaagcaca agcgcatcat gctgggcttc     300
   actcaggctg acgtggggct ggctctgggc acgctctatg ggaagatgtt cagccagacc     360
   accatctgcc gcttcgaagc tctccagctc agctttaaga acatgtgcaa gctgaagcca     420
   ctgctgcagc gttggctcaa tgaggcagag aacacggaca acatgcagga gatgtgcaat     480
   gcagagcaag tgctggccca agcccggaag agaaaacgca ggaccagcat cgagaccaac     540
   gtgaagggaa cgctggagag cttcttccgc aaatgtgtga agcccagtcc ccaggagatc     600
   tcccagatcg ctgaggacct caacctggac aaagacgttg tccgggtctg gttctgcaac     660
   cggcgtcaga aaggcaagcg gctgctgctg ccctttggca acgagtcgga gggggtgatg     720
   tacgacatga accagtccct ggtgccccct ggtctgccca tcccagtgac atcccagggc     780
   tacagcctgg cgccgtcccc ccccgtctac atgccacctt tccacaaggc cgagatgttc     840
   cccccgcctc tgcagcccgg gatctccatg aacaacagca gccactga                  888
<210> 3
   <211> 51
   <212> DNA
   <213> Artificial
<220>
   <223> Interfering RNA
<400> 3
   aagatgttca gccagaccac cttcaagaga ggtggtctgg ctgaacatct t 51
<210> 4
   <211> 51
   <212> DNA
   <213> Artificial
<220>
   <223> Interfering RNA
<400> 4
   aacagaaacc ttcaggctgt gttcaagaga cacagcctga aggtttctgt t 51
<210> 5
   <211> 51
   <212> DNA
   <213> Artificial
<220>
   <223> Interfering RNA
<400> 5
   aaggccaaga gccgcacagc tttcaagaga agctgtgcgg ctcttggcct t 51
<210> 6
   <211> 51
   <212> DNA
   <213> Artificial
<220>
   <223> Interfering RNA
<400> 6
   aacgcagttc atagcagtta cttcaagaga gtaactgcta tgaactgcgt t 51
<210> 7
   <211> 47
   <212> DNA
   <213> Artificial
<220>
   <223> Interfering RNA
<400> 7
   gcggcaaagc cgacaacaat tcaagagatt gttgtcggct ttgccgc 47
<210> 8
   <211> 51
   <212> DNA
   <213> Artificial
<220>
   <223> Interfering RNA
<400> 8
   aattggctca tcctggatag attcaagaga tctatccagg atgagccaat t 51
<210> 9
   <211> 51
   <212> DNA
   <213> Artificial
<220>
   <223> Interfering RNA
<400> 9
   aagggcttca gagacaacta tttcaagaga atagttgtct ctgaagccct t 51
<210> 10
   <211> 12115
   <212> DNA
   <213> Gallus gallus
<400> 10
   ggtggaattc gaaagaggaa gagtgagaca ttcaaagtgg tggtgtttgt ctccccagca 60
   aactgttaca tgagatgagg cctgcttttg tgtaagtggc tgagcagctg cccgccaatg 120
   gaaaatagtg aatgaattct ttgttttgct ttgattgcat gcacagcttt cacctagcaa 180
   actgtatctc ttcctacaag atctagcact ttcacctttc cgattctctt ccctctggga 240
   agagcaaaca aacaactgtg tggtgctgag ctgcctgttg gattatacta cagcagtaac 300
   tgagtttgag tgcaaatttc agtccacagg aaactaagat gtcccatgcc caatacaggg 360
   aacagaaaag ccacagtaca taaagtaata gcagtgacca taaagagcct acatgtaact 420
   ggcttcaggt cattttcatc ttctctgttc ataagaacaa ggcacccaag caaagaacac 480
   aacccccaag tttctagatc ttttccacag cctcatatgc atatgtcaca tggtcaaata 540
   gcaattgtct agttacctat ggaagcaaca agagtattct catgtttcat gaattgattt 600
   ttgtctctga cagtccaaga gagattgatt ctgattccta aactgtaatt tctttgttac 660
   tcttgagaaa tattactcag gtgtaccgtt tatacttgga ctatcagatc tcatttaata 720
   tgtttatttt tgctattaca ttagatacag cattacattt tctattttat ttccttgttc 780
   acccctcaca cactgggcac agaagaatct gtcaggaact gaactgaata tgatgtcact 840
   tcttgcttac tgttcgacca cagattgatt tacacagcaa atacaactca aaaagaatgg 900
   cttgaaatag aaaattcatc atagtattat agataatcag aatatgaaca actgattcag 960
   aatccagagc cactgttttc tgtgaccaag tcacagtagg ttttgccaga tagtactagg 1020
   ttatagcgag ggtcagctac tccctagggg atgagaagtc atgtgcccaa agtagaactg 1080
   ttgcatgttg aagccaaatt accacctaag tgcctgacat ggaaaccggt gggaaagaga 1140
   tccatgtttg agaccagcct cagctttgct gactcacatg agtggtgact tgagttggtc 1200
   tctgtgtgtg catgtatgca tgtgtgtaaa gcttttcctg atatccctca cttgattcac 1260
   ttcataaatt gcgcttagag gtgtcttatt tctgctgttc ttcttccccc cataacaacg 1320
   aatgtgatcc attctctctg cttctttaat acaatcttga aagcaccagt tcttgttttc 1380
   ctaaggttga aaaatctgta tgtgatcaag gggtgcatcc caacaaactg gattttactt 1440
   aacagaatag tgctactgtt ctgtgagtta caggtagtca tctttcagtt tctgtaaagt 1500
   caaaatgtct ttaggttata tctgtccata actctgagta attcttttga ctgtgttaaa   1560
   agattagtca tataaatgag agaagaatca gatccaattg tagctttaga gtttaatgac   1620
   tgaaatgggc aataaaaaat gaatatttat tttagtcttg caaaaataac cttgccttgg   1680
   gaaaagcaaa cacagaaaga ttatattccc aagcacatag tacagagagc agggctgtac   1740
   agatgacagt acagaggaag aagagtagtg ggccttggac actgttcttc attatgcctg   1800
   ctacctgcct ccatttccac atcctgaggt ttctcatcaa ggatatttag catggtccag   1860
   tgtactcaag accctcagtg tcaagctgac tgtgcacaat attggtaaaa agtgaggaaa   1920
   gttggcaaca ttttatgact tctgtcttct aaacatagta aagtagatgt ccattctctg   1980
   tggaatagat aatcctgaaa caaaaactaa aatatgacat ttagatcctt caacagttac   2040
   actttgaatt tgctgtgatt taaaatggac atagcatttg taatagcaca gaatatttcc   2100
   tttccactgg tgtcacaaaa agtggatttc ccagatgtgc aatgacctac ttaactataa   2160
   atttatttca ttccatttca ctaaaaacaa catgtcagag aactacttag tcagattttt   2220
   tgtttggttg tttggtggtt taaaaaaaaa aacaccaaaa acccaagggt gtgtgcttct   2280
   ttgcaagtcc aaagatctgg acttattcac tgaagtagag cctctcatat ccaggggcac   2340
   aatcatttag caccagcatc ttcaggaaca caatcactct gctctttcgt gccatttagg   2400
   aagcagccta agcatctgat ctgtgcaatg ggcctacatc cttgcaatgc acagcatgag   2460
   ccatgctcag taccacctgc aatgccagtg acgcatatgc tgacaagaaa tcacagctgg   2520
   gggcagtctg cagtgtcctg tctggatgtt gccatgggcc cacacatgag ctgggacata   2580
   tctgtgtttg tcctgagttt taagacacag taaaatacaa cataagctag gaaagctttc   2640
   tcttgacttc aaaaagcatc accatgatga tttggttttt aatctgactc acaactgaaa   2700
   cagggtccct ccatcccttt cctgccaaaa tttcactgat tttttcacct acatactgta   2760
   ttatcctcca aaacaactaa tagtttgtag ctttcagctg gaatgtcata ggaccaggtt   2820
   taaaatcata gaatccttag agttcaaagg gacctctgaa agccatctag tctaactctc   2880
   ctacaatgaa cacagacaac acagccagat caggctgatc agggcattat ccaccctcac   2940
   cttgagagtc tccagggatg gggcatcaac cacatctcta ggcaacctgt tccagcgcct   3000
   caccaccctc actgtaaaag attttttcct tatatctaac ctaaatctaa cctccctgag   3060
   cttgaaacca tttttccttg tcctgtcaca acagaccctg ctaaagagtc tgtccccttc   3120
   tttcctgtag ctccccttca gatactgaaa ggccactatc aggtcacctt ggagctttct   3180
   cttctccagg ttgaagacac ccagttctct cagcctgtcc tcacagaaga ggtgttccat   3240
   tccatggatc atttttgtgg cccttctctg aacatgctcc aacaggtctg tgtctctcct   3300
   gtactgagga ctccacatct ggatgcagta gtccaggtga ggcctcacca gtacagagca   3360
   gaggggtggg atcatcaagc ttccctggct tctttggctt cttgccaggg ttgattctga   3420
   gtaaggggaa gaaaatgtta ggtaaatcat gttatgtggc tctcttgtcc aacatgcaga   3480
   caagacagca gaggagcatg acaagtagta gatgggaagg aaacccttct cccatctgag   3540
   ctgaaggctt taattttgtt cgggttcaca atactttccg gatgtccact ggatgtatct   3600
   cttctctcag gaatagcctt cagcagccaa tcctattggg aacacacagg aaaaaaagag 3660
   agaagaacaa aggaaggaag atcataagac tcaagatttt attcatgtga gaacagttga 3720
   aaatgctatg agatgtgctc cttcttccta gaggagactt catttgtctt cttcattttt 3780
   atgacattta atttgtcctg tccttcactt gtagttcaat ttgagtttat acatccttga 3840
   aagagtcact tgtatgtgga aagtgagggc caactttgtg aagatctctc cagtttaagc 3900
   ctgtcccacc gtatcagtat ggagcagatg ttacagagat ttgtgtcttt ggtgccttgc 3960
   tgtggtgtac aagcaatctg gtgatcaaga ggtcactgga gcaggaagtg agtatagaga 4020
   atatcctgag aggagaagtg gtgagacctc acatacctta gccactgttg tcatttagca 4080
   aatacagctc ttctttgtga agtcaccagc ttgagatctc ctggagacta ccattcagaa 4140
   atgggaagaa aggacacagc aacattgagt gtttctcagc cccttgtttc aagagacact 4200
   ggtgcattag gagtgaacct ctccaatcct gggttttttg tgaaggaaat gactagcatt 4260
   tttttcacta tgtgccagtc aagtgggtca tgctccatac acagccttga acgtggctcc 4320
   ccatagctct agggttatac tgaggtctct gtacccaggc ttgtgcttca tctcaagagg 4380
   tgcaggaaga ggtgcaggac atcctacctc cacctcatct agctatctct gctttttcca 4440
   gtcccttgat tttcctctcc ccatgacttc acctctctcc cagggccaca gaccagtgaa 4500
   gctgaagcac aactctgttg cctctccagt agaggccagg gggagtgggc aggcaggcaa 4560
   tacagccccc aagacacgac tgccacaagc tcagtggtgg tcatagcagg gcagtggtga 4620
   ggcagcccta ttgcttgaca ttactttgat tatatcattc agcactgcga ggaacatgtt 4680
   taagtgctcc tcatggctgc ctcaagtcat ttccatgaaa gacatgttca ggaagcaggc 4740
   attgaagcca gtagctggtg tacaacctat tctctggaga tcaaaacata gaagaggcat 4800
   gatccagctg cttatgcaac aggagcaggt cttaatcact gagttgtgct gggaaacaca 4860
   cagcctaatg tcctgctctt gctgcagggc gccaatactg tctagtcccc atagtgggtg 4920
   catctagtgg gcagatcaga aatcaccacc agctttggcc agcacatggg attgcagagc 4980
   aggtgggtac tgaaagaaac tgaaatctcc agtggtttat tttaactcac tgtctgtgga 5040
   ctgcagtggc tgagaagaga ttaactatat cctgctggac catttctaca gcagcagtgg 5100
   gaacatctgg cagctgcagt aaatgaagaa agataactga ggtgttaagc ttgtcagagg 5160
   gccctgctgg atcccaggcc aagacctgta tattcacacc aaaagtatgc aacactctgc 5220
   aaggttactt tgcatctgca gatgctttga cactattaat agattgttta caactatagt 5280
   ggcatgattt agtcatgagt gggattcaga taaggtgttc tcaggcctgt cttagcccca 5340
   gagtttgcat gcaaacattt cacagctgac ttcagaactg gaatttttag caagatgttg 5400
   ttcccaatag tcgtggctgc tcctcctcct ccttcatgac taagttgtac tgaggaagca 5460
   tttttatatt ctcacagatg agtttgagtt tctggagttc tgctattgta tgattctgtg 5520
   ttttttcctg atcttgcagc ctgggaagac acccagtcaa atacgcagtc tctatttctt 5580
   tgctgctcac aaagatgtgt tttcacttcc tgatgtaaaa agttcactct ggacaattcg 5640
   aaggagatga acagatcatt tttacctttg ttgatttgaa gaagcaggcc acaatatatt 5700
   ttgtaatgta tttttagaaa taattgattt ggaaaagtag atatgccata tatttcagta 5760
   gtgtattttt agaaatatag ttgttgaact gtgttatgtg attagttctt gcttgcaaaa 5820
   ctgtaatagc ttttagatgt tgtgaatagt aagaatgtag cattattaac tagaaagcat 5880
   gttgtaaagc tattctgttt tgacaagaga ccctcagcaa aggagacaca ataatgaaag 5940
   ccagggcctc cacacaaggc tgaattgtct cactctgcaa ttaggttggg atgcaacacg 6000
   cagacattaa gatactcccc tctatcctcc ttatcttcaa caacctagta tatatcatta 6060
   aaagacagag tgccaaactt atctcgatcc aaggatgaac aggtgtccag aaataatgtc 6120
   tgagtgctga gagagcgaat gttacgattt tggtacctat aaattactta taataggcat 6180
   tctcctactg agcatgcatc aatggagaaa gttcatctag atgacaagca aggaagacca 6240
   ttggatgaag taaaagactg tctcttggac tgtcatgatg ctaaaaggat gctagacagt 6300
   agaaaagacc atggagtaaa aaactggaaa cctcataaat cactacctga gataggcgtg 6360
   tataattgtc tcacgcataa ttgatgagta tgtactatcc gtggaaatat aatggattct 6420
   gaatcatgta atctttgaag cacttatggt ggaaatatcc ccagagtttc ccagtgtcac 6480
   taataaagaa tacctgactt aacagtaaca aaactttgct gttaagtttc tacacatcga 6540
   tttcttcaat tcaatgtaaa tgttatatcc tcttcccaag cctccaactt ccactcaatt 6600
   cagttcatag aatggcttag aatggaaggg atcttaaagc tcatccattt ctaacaccct 6660
   gctgtggaca ggattgccat ccattagctc aggctgcaca tcgtcccaat tcaactcggc 6720
   cctaaacacc tccaggaacg ggccatccac aacttttcag gacaacctgt tccagcgcct 6780
   gaccatattc tgagtaaaga atttcctcct aaaagctaat agaaatcttt cctcttttaa 6840
   tttaaaacca tttcctcttg ctctccctct atcagactgt gtaaaaagtc tgtctcgatc 6900
   ttgtttataa gcttccttta agtactaaaa agcaattcct aaataaaaga cattctgtta 6960
   aaaaaaaact actgtcgctc actgttcttc cttttgtctc tttatctttc ccatctcagc 7020
   aaagtcggcc acataaatat tgttagacaa aagcaggtaa atccttttct tccgtcagtc 7080
   ccagacaata tccacttgca gatcccatag aacagcactc ggtttcaccg gctgccattc 7140
   agatgtagag cgggtgccag ccagggagcc tcaggctcta acagttgcta atcacacatg 7200
   aatgtgtgtg caagagccag tcttggagga aaggagacgg tggaccaatg gcggtaagga 7260
   ccagacagtg tggaggtctc tgtccctacc tgtgagaaat ttgaaagaga agaacccttt 7320
   ctcagtcatc cgtgtaacca gacacttgcc ttataggtga gctgctcctg tggggacttt 7380
   ctgggcttca gaaatccaga tgtaacagaa tgggcacgat gaattgctcg agaagtatgt 7440
   gctaattggt tcatgagata tattaggaat aaaatgactc ttagatggtg tgcctactta 7500
   tggcctgcac aagcataatt acagtcatcc agttggcacg taatccccat taacaacact 7560
   gaaaaacgtg ggattagctt cggatgaaag atgtggaaga aaacaggtgc cacaatacac 7620
   agcgaaccca agagaacatt caacgcacat cctaccactc aaatgcccca ctaactgtag 7680
   gctctgagta gctatggact ttgcagacac ttttcccact gaatgggttc tgtaaaattg 7740
   tccccaccag aaagagtaaa gaaacctgaa gaaaaaaaaa agcctctgtt tcatttaaaa 7800
   gtttacaaaa ggaatctgtt cagggaagaa atcacaaact gtctgataca gagcatcctt 7860
   taggaggaga gcatgggagg tgtcacgtat ttttaaatta cacggggtct ttgtacgacg 7920
   agaaatacag cactaggtat ccccccctgg tggtggaaat ggcagaaact ttcaacaaag 7980
   caagcacagc catcactgtg catttgtttt tagtaagagg acatctaact gagcctatga 8040
   gaacaatcac tgttgtcaaa agggagaggg gagaagttga tcagggagcc catttggaag 8100
   gttcttgtcc aatacgcagt ctctacatgt tccccagtac tgaacaaccc tggctctcaa 8160
   gtactcctca gggagatcat tctttattcg agttcataag cactgatagg aaatgctttc 8220
   tgagacatgt acttgcaaat acagcacttc agcatttctg tgtgtagcaa gcattagtgt 8280
   aaagaagggc tgataaagaa caggcccatc tgtgttgtct cactagcttt ctttccacct 8340
   tattttgaca ctccagattt tctgccggcc tgcctccagg ttttatgcca cggttcctct 8400
   gttcaagtct gcctctccac ggctaacaat gagtcaacca tcaaccagta attctcaggt 8460
   gattaagcag cagggttgtc agtggaaagg agaagctttg tccttttctt gacctgttcc 8520
   cctaaaaagt caagagaaag aaaagctgta tttcaatatg aatgaaggtc taatttttat 8580
   tattctttgt tggctgcctt ccttccacaa acatgacatt tttgatccag actgctagct 8640
   gtgaccaagc catgccgggg tggctgtctg gagtcaaggg ctgtggaaca gatgttttct 8700
   gaaggttaac tgaagagagt tcaacctaaa aaataccaag ggagactgct tgcaggaaaa 8760
   ggtggacaaa ctcaagagaa tacaatgaac ccatttcttc ctgcatttct tcttccccca 8820
   tcatgcacat actttacaga gcatgatggc tcccacagaa acctctgagg agtaacaagg 8880
   ctcagccatg ggcccctcgt tacagctcac tgagcaccat gaacatgaac aatacaggaa 8940
   ctctcagtga cacttcaaaa tattcactaa aacacagatg agtcacgttt gagtcagttg 9000
   cagaagaaca cttaatctac ataaaatttg agacacctct accagcataa cagtgaccca 9060
   ctgatgaaaa tgaacttagc agtgctacga ggtaggaacc catagaactc tgatccagca 9120
   gtacaagctc cgaagctgaa tttacagtca gctgctgctt ccccagcccc tgtaaactgg 9180
   tctgtctgta tcactttagt gtaagtttga gctccatccc tctgaaagcg gtgaccctac 9240
   ttacagtagt aagtgaaccc tcgtagaagg aacttgcatg aaaacacctt ccagcccttg 9300
   tccaatctca cctgtgtttc agccaagaga gcttttacac atcagcttct accctggaag 9360
   gactatggaa aaggctcctt gggacatact tctgtcaagt gactgcttac attcccttcc 9420
   ataggcttaa tgacccagct gatgcttaca cagtgactaa tgctccataa tattaggcac 9480
   ataatgaagt gataactgca aaagcaggga gtggccacag cattaagagt gaggtgacac 9540
   agccttcagc ttaaaaacaa atcaatttca atctgttggg aagaatagga ccaaaacaga 9600
   tctgaattgc gttgctgctc cacctgaaaa cagcagcaaa aaggaatatg agcgaatatt 9660
   tggatctgct ctcagagtca ttctcagcag gtgtttttaa aaaatcatca tctctaaata 9720
   ttaatgggca gtttatccac tttgcagctt aatggtgtca cagggcatat gtcccagatc 9780
   cagcacctca ggaagcctct tgaatgtcta catcctgctt cctatagcat tctctgggcc 9840
   tgtatgactt atggatttac cttctcaata tgttattatg tgtaaaattt attttaggga 9900
   aaataactga atttcttttc ataaattctg actttagcgc tagacctgat tttatacact 9960
   cacacaagcc ttatttctcc tataaagaca tttcaaaaga taagtgataa tttggtaaga 10020
   gtcctgactg aaagcaaact gatgtcacta gagggcgaca cgtggaacaa aaaaacagta 10080
   aatataaagc aaattagaga caagtgagat gagaaaatca aaagataaac aaagattgtg 10140
   ggaaaattaa ggggaaaaaa tgaaatcaat tccatgcaga cagcaaagca ggtccatctc 10200
   tgggagggaa acagctcaac agcaaaaaaa ttataaacca agggagactg gggatttgat 10260
   ggcagatgag gtgttagagg ctgtaaaatg ctaatccttc tgctatgggt gcccctagac 10320
   aagctgtgtc acctctgatg actcagtctg cagcttgtgg gatggcaaga gtgcatcgtg 10380
   gaacttctca gcatcgtgtg gcatggcctc cagtcttctg tctgtgtcaa tagtatttac 10440
   tatttcactt aatgcgcact gtagaggcaa acaaatggag ccccttaagt tattattgcc 10500
   aagagacaaa caagtgcttt ggtttgcaaa cacaacttcc ctgacattga aatcctgtgt 10560
   ttttccctct ctttgcaata ggctgttttt ctatgagctc agacattgtt tcacaggtag 10620
   cacacatatc tccagggaag ccctgtcagc aaagggactg tgcagatcag tggccaccac 10680
   aaccatatcg gttacatggc aattttgcac atttgacaga taaacccttt ctctgtgtgc 10740
   attggctaag cccataacag caagcaagca accgtgtgtg aaaccaacag ccgaacatgt 10800
   ggggacatgg aagagcctac gggaaacgga acgcacagct tacaccacgt gcttcagggc 10860
   aagacagcat ctgcctccac attgtacccc tcatcgaatt ataataactt gctcctgcat 10920
   ccctaatcac ctctctacct ggaccctacc cagattctga gaaactaaaa caccataaat 10980
   tataaagtca taaaaaatga gctcaatgag ggcccctcca agttgcaaaa gcaccttcag 11040
   gtcagcaact ccctgccact acctagcaac tctggagaaa gggggcttaa cgtaacacat 11100
   gggggtgtct gctcatcatg actaagctgg ggagggagaa atatatggaa gtaagggggg 11160
   cctatttctg ttgcctctag aagattatta gcagctaata tctgttcctc atttcttttc 11220
   agacagggag gagcctgtgt cctgtccttt cactaggtga agtagggaat ttgcttgatt 11280
   tgtcatatgc tctcatttgt ggatctctca ggttgttttc tgcattttat aacccctttt 11340
   ttgtaaactc atgcttattt caaatgtcat gaaaagaatt gacaaatatc tgaggaaaga 11400
   tgctgagaag tgacacttct ttgtgctcct ccaaaataga tgtctgtctc atccttgggt 11460
   tagcatttag aaaacctggg gagctcgtct ttgagtttgc ttccctgtga tgcagacacc 11520
   atcctagaat accgccctcc ccgccggccc cagtctgtat tcccttgcaa ggaggaatgc 11580
   ttatctgttt acaatgatgg ctgcagtctg caatgcagac acgaaaagcc tccgcatgtc 11640
   ccgggggggt ctctgggctc cccctccatt ctttgtactt gggtggggac cgatgaggag 11700
   gcgtaggtgg gtagcaagac tgttaaatgt gagggcgggg gtgccagccc agcccgccgc 11760
   acgggacggc cttgctcaca cctgctaggc ctgaggtgga aacaaggccc cctcctccca 11820
   gccccggggg gtcgggtgtg cgggcagcaa agaactgact cccaccgagg aaccaaaggg 11880
   ggaagctggc cttgctgtcc tagtcctccc ccactctccg aatatcccca tagccactcc  11940
   cctccaatca actgactttt aacatgataa tggtcgtgac aatctcttgg ctaaagccag  12000
   tctggggagg acataaaaca ttgcccctca ccgcctaatg gggtatctcc gtgcagtagg  12060
   cgcgtatgca accagctcac cgccagcaac ggctggaggt gctgtcccga ccatg       12115

## Description

La présente invention concerne un procédé de préparation de cellules aviaires différenciées, à partir de cellules souches en culture. Des gènes impliqués dans le maintien de la pluripotence des cellules souches aviaires ont été identifiés et clonés. En inhibant l'expression de ces gènes dans les cellules souches, celles-ci perdent leurs caractéristiques de pluripotence et s'engagent dans une voie de différenciation. Ces cellules différenciées obtenues *in vitro* peuvent servir de cellules hôtes pour des pathogènes, notamment des virus, et être ainsi utilisées pour la production de vaccins anti-viraux.

Une cellule souche est une cellule pluripotente ou multipotente d'origine embryonnaire ou adulte qui présente une capacité d'auto-renouvellement. En d'autres termes, une cellule souche est une cellule non cancéreuse capable de se diviser indéfiniment en culture et de donner une cellule fille présentant la même capacité de prolifération et de différenciation que la cellule mère dont elle est issue, et qui est capable de donner naissance à des cellules différenciées.

Les cellules souches embryonnaires de poulet (CESC pour Chicken Embryonic Stem Cells) ont été isolées par la mise en culture de cellules de blastodermes de poulet au stade X (Pain et al., 1996; demande de brevet N° FR 94/12598). Ces cellules CESC présentent toutes les caractéristiques de cellules souches embryonnaires (ESC). Un milieu de culture permettant de maintenir le caractère pluripotent de ces cellules d'oiseaux a fait l'objet de la demande de brevet WO 96/12793. La plupart des caractéristiques de ces cellules sont décrites dans la publication Lavial et al. (soumis).

Une des propriétés des cellules souches est leur capacité à donner des cellules différenciées à la fois *in vitro* et *in vivo.* L'obtention de ces cellules différenciées à partir d'une population homogène de précurseurs est réalisée par la modification des conditions de culture. En effet, les cellules CESC ne demeurent en prolifération dans un état non différencié que dans des conditions définies de culture *in vitro,* en présence de facteurs de croissance et de cytokines ainsi que de 'feeder' inactivé. Dès que le milieu est appauvri, l'équilibre de prolifération est modifié et la cellule s'engage en différenciation. Un des avantages de l'obtention de cellules différenciées *in vitro* à partir des cellules CESC est la très large palette de phénotypes qui peuvent être générés à partir de ces cellules. En effet, les cellules CESC présentent des propriétés de pluripotence ; elles peuvent se différencier en tous les lignages définis au niveau embryologique, soit en dérivés mésodermiques, ectodermiques ou endodermiques. Cette propriété de pluripotence, spécifique d'une cellule souche, n'est pas présente dans les cellules primaires d'un tissu qui sont déjà engagées dans une voie de différenciation. Cette propriété est maximale dans les cellules souches embryonnaires et est présente mais de façon plus restreinte au niveau des cellules souches adultes tissulaires, qui sont multipotentes. Ces dernières ne peuvent se différencier que dans des voies de différenciation appartenant à un seul et même lignage.

Les processus de re-programmation nucléaire permettent à une cellule différenciée de retrouver une plasticité de différenciation. Ces mécanismes commencent à être décryptés au niveau moléculaire notamment par la compréhension des modifications épigénétiques qui se produisent au niveau de l'ADN (méthylation sur les ilotsCpG) et /ou de ses composants comme les histones par des processus d'acétylation et de déacétylation, de méthylation et de déméthylation, d'ubiquitination des lysines, de phosphorylation des sérines, d'isommérisation des prolines et dont une des conséquences est de recruter des e de nombreux complexes protéiques qui contrôlent par leur association sur des promoteurs le niveau d'expression de gènes clefs. Des modifications post traductionnelles directes de ces acteurs sont également un élément de régulation supplémentaire.

Différents procédés sont connus par l'homme du métier pour induire ou contrôler la différenciation des cellules souches pluripotentes en cellules différenciées *in vitro.*

Une première approche consiste à ensemencer les cellules dans un milieu de culture contenant peu ou pas de cytokines et facteurs de croissance nécessaires au maintien de la prolifération des cellules. En particulier, l'absence ou la faible concentration d'une des cytokines de la famille gp130 (LIF, IL-6, CNTF, GPA, IL-11, ...) induit un ralentissement de la prolifération et une perte progressive des marqueurs de la pluripotence.

La différenciation des cellules soumises à ce procédé n'est pas homogène ; différents types cellulaires seront obtenus selon les conditions de densité, de sécrétion autocrine et paracrine des cellules, et de leurs relations les unes avec les autres. L'hétérogénéité des cellules obtenues peut être estimée par la détection dans l'ensemble de la culture de la plupart des marqueurs précoces de lignages, comme par exemple les gènes *Brachyury* et *Goosecoid* spécifiques du lignage mésodermique, certains gènes *Pax* et *Sox* spécifiques du lignage neurectodermique, et *Hnf3* spécifique du lignage endodermique.

Une seconde approche consiste à réaliser des corps embryoïdes par ensemencement des cellules dans une boite qui n'est pas traitée pour la culture cellulaire. Les cellules dissociées sont mises en suspension soit dans un grand volume de milieu appauvri (moins de sérum - de 0,5 à 5% par exemple - et une absence de facteurs de croissance et de cytokines spécifiques) et soumis à une agitation lente, soit dans une petit volume de milieu appauvri qui est alors utilisé dans la technique des gouttes pendantes. Différents exemples sont donnés dans les brevets US 5,456,357; US 5,914,268 et US 6,458,589. Un autre conditionnement est la formation des corps embryoïdes directement dans un tube conique (Kurosawa et al., 2003), sur une surface traitée particulière (Konno et al., 2005) ou par encapsulation dans des microbilles d'alginate (Magyar et al., 2001). En empêchant ainsi l'adhésion des cellules et leur polarisation baso-latérale, les cellules souches embryonnaires prolifèrent et adoptent une structure tridimensionnelle qui mime les différents feuillets embryonnaires (Dang et al., 2002). Les types cellulaires ainsi obtenus sont très hétérogènes. De plus on observe une hétérogénéité dans la cinétique d'obtention des différents stades de différenciation.

Une troisième approche est l'utilisation d'inducteurs chimiques de différenciation. Par substance inductrice chimique, on entend toute molécule chimique non peptidique ne s'apparentant pas à un facteur de croissance ou à une cytokine, qu'elle soit d'origine naturelle ou obtenue par synthèse chimique.

Par exemple le DMSO (Diméthylsulfoxide) est utilisé comme un inducteur général qui permet l'obtention de populations différenciées mixtes avec plusieurs types cellulaires dérivés (Dinsmore et al., 1996). L'acide rétinoïque permet, seul ou en combinaison avec l'AMPc par exemple, de façon non exclusive, d'obtenir la différenciation des cellules souches notamment en dérivés mésodermiques, avec l'obtention d'adipocytes dans des conditions de cinétique précises (Dani et al., 1997), de cardiomyocytes, de différentes cellules musculaires caractérisées par une activité contractile et la présence de myosine spécifique (Rohwedel et al., 1994 ; Drab et al., 1997).

La plupart des protocoles qui sont actuellement publiés et qui permettent l'obtention de cellules avec un phénotype particulier à partir de cellules souches embryonnaires de souris associent des inducteurs chimiques et des combinatoires complexes de facteurs de croissance. Des variations dans les cinétiques d'induction et de mise en présence de ces agents permettent de moduler les phénotypes obtenus. Des exemples sont donnés avec la cinétique d'obtention de cellules adipocytaires (Dani et al., 1997), de cellules ostéoblastiques (ZurNieden et al., 2003 ; Philips et al., 2001), et de précurseurs neuronaux présentant différents phénotypes (Fraichard et al., 1995 ; Plachta et al., 2004 ; Glaser and Brustle, 2005).

La reproductibilité de telles approches est parfois difficile et ne permet pas d'obtenir des enrichissements en cellules différenciées satisfaisants pour des applications industrielles, comme la réplication de virus particuliers ou la production de molécules d'intérêt. De plus les processus comprennent de nombreuses étapes, impliquant l'utilisation de milieux de culture bien particuliers et de nombreuses étapes de manipulation des cellules.

Les approches décrites précédemment permettent d'induire la différenciation de cellules souches ; les populations obtenues étant hétérogènes, il est ensuite nécessaire de sélectionner et d'isoler les cellules présentant le phénotype souhaité; pour cela plusieurs techniques sont connues de l'homme du métier. On peut citer de façon non exclusive le tri par trieur de surface suite aux marquages par des anticorps de surface spécifiques, par déplétion ou par enrichissement magnétique.

Une autre approche consiste à utiliser des protocoles d'enrichissement qui font appel à des processus de sélections génétiques positives. Pour cela l'expression d'un gène de sélection d'une drogue (néomycine, hygromycine, zéomycine, blasticidine) ou d'un marqueur phénotypique permettant le tri physique d'une cellule qui l'exprime (protéines fluorescentes comme les GFP sauvages ou modifiées, beta-galactosidase, alcool deshydrogénase) est placé sous la dépendance d'un promoteur tissu-spécifique. Parmi les promoteurs utilisés on peut citer ceux des chaînes de *Myosine* (Muller et al., 2000), ceux des gènes inducteurs de la myogénèse *Myf5* ou *MyoD,* ceux de la *Nestine* (Keyoung et al., 2001), de *Hb9* (SinghRoy et al., 2005), de promoteurs spécifiques des astrocytes comme la *GFAP* (Benveniste et al., 2005) ou d'autres types neuronaux comme les oligodendrocytes avec les genes *CNP* (cyclic 3' phosphodiesterase) (Glaser et al., 2005; Schmandt et al., 2005).

Une autre approche consiste à sur-exprimer le cDNA d'intérêt capable d'induire la différenciation dans un lignage donné, comme par exemple avec les cDNA du lignage myoblastique (gènes *Pax3, MyoD, Myf5, Myognénine, Mft-4,* ...). Cette sur-expression peut être réalisée à l'aide de vecteurs de sur-expression à insertion aléatoire, mais aussi à l'aide d'une stratégie de knock in dans un locus donné de l'actine ou dans le locus spécifique du lignage, ou encore à l'aide de vecteurs viraux et rétroviraux.

A l'heure actuelle il n'existe donc pas de méthode permettant d'obtenir une population de cellules différenciées *in vitro* qui soient homogènes, sans réaliser ultérieurement une étape de sélection. C'est pourquoi les inventeurs ont mis au point une nouvelle technique permettant d'obtenir une population de cellules différenciées présentant des caractéristiques morphologiques, biochimiques et fonctionnelles homogènes, qui ne nécessite pas d'étape de sélection supplémentaire. Selon l'invention, il est possible d'obtenir une modification morphologique, phénotypique et moléculaire de 80 à 100% des cellules d'un clone induit, c'est-à-dire d'un clone composé de cellules différenciées.

Chez les mammifères, plusieurs gènes contrôlant la pluripotence et les capacités de prolifération des cellules souches ont été identifiés. En particulier le gène *Oct4* (Nichols et al., 1998) est un gène clé exprimé uniquement dans les cellules pluripotentes *in vivo. In vitro, Oct4* est exprimé dans les cellules souches embryonnaires de souris et de primates, ainsi que dans certaines lignées cellulaires tumorales. Le niveau d'expression de *Oct*4 dans des cellules souches embryonnaires murines semble contrôler le devenir de ces cellules souches (Niwa et al., 2000).

Le gène *Oct*4 appartient à la famille POU des facteurs de transcription à homéodomaine; la protéine produit du gène a une localisation nucléaire et peut se fixer directement sur les éléments de régulation des gènes cibles via son domaine de fixation à l'ADN.

D'un point de vue phylogénétique, il était peu probable qu'un gène de fonction équivalente existe chez les espèces non mammifères. Chez les oiseaux, un tel gène n'avait pu être identifié lors de travaux précédents (Sooden-Karamath & Gibbins, 2001). De plus aucune homologie au niveau génomique n'a été reportée.

Chez les mammifères, les gènes *Nanog* (Chambers et al. 2003: Mitsui et al., 2003; Hart et al;, 2004) et Eomes (Russ et al., 2000, Ginis et al., 2004) ont également été identifiés comme jouant un rôle clé dans le maintien de la pluripotence des cellules souches.

La présente invention propose un procédé de préparation de cellules aviaires différenciées à partir de cellules souches aviaires cultivées dans un milieu de culture approprié, caractérisé en ce qu'il comprend une étape d'induction de la différenciation des cellules souches par inhibition de l'expression ou de l'activité du gène *Nanog*.

L'avantage de ce procédé est que les cellules différenciées obtenues sont homogènes. Les clones obtenus ne présentent plus la morphologie caractéristique d'un clone de cellules souches, mais une morphologie différenciée. Ces clones ne présentent plus non plus la prolifération caractéristique des cellules parentales et la cellularité reste constante après l'induction.

Cette induction de la différenciation des cellules souches est à la fois rapide et irréversible.

De façon préférée, les cellules aviaires sont issues d'un oiseau appartenant à l'ordre des Galliformes, en particulier d'un poulet ou d'une caille, et plus préférentiellement de l'espèce *Gallus gallus.*

Par « cellules souches », l'homme du métier comprend que les cellules présentent les caractéristiques suivantes :
- la capacité de proliférer en auto-renouvellement *in vitro* en présence de facteurs de croissance bien connus dans la littérature, et pendant des temps importants ;
- d'un point de vue morphologique, une cellule souche est caractérisée par un rapport nucléocytoplasmique fort, une taille relative de 10 à 15 um avec un noyau de 5 à 10 µm, présente différentes activités biochimiques intrinsèques comme celles de l'alcaline phosphatase et de la télomérase, et est reconnue par différents anticorps spécifiques tels que décrits dans les brevets FR 94/12598 et FR 00/06029. Une autre particularité des cellules souches embryonnaires aviaires est leurs contacts membranaires étroits quand elles prolifèrent ;
- les cellules sont pluripotentes ce qui signifie qu'elles peuvent donner naissance à plusieurs types cellulaires, en particulier les cellules souches aviaires de l'invention peuvent se différencier en cellules de dérivés ectodermiques, mésodermiques et endodermiques.

Par « cellules différenciées », l'homme du métier comprend que les cellules prolifèrent moins ou plus du tout, qu'elles présentent un rapport nucléocytoplasmique plus faible, une taille en général plus grande (> 15- 20 µm ou davantage pour certains types cellulaires particuliers comme les ostéoclastes, les adipocytes matures, les cellules musculaires différenciées, certains macrophages, etc), avec une morphologie moins compacte et qu'elles ont perdu les marqueurs biochimiques des cellules souches non induites.

En particulier les cellules souches peuvent être différenciées en cellules épithéliales, caractérisées par la présence de marqueurs comme les cytokératines 8, 18 et 19 ou la kératine 16 pour les keratinocyte, certaines mucines, cadhérines et intégrines spécifiques. Selon l'origine des cellules épithéliales considérées, différentes associations de marqueurs seront caractéristiques de l'origine et du degré de différenciation des cellules épithéliales, notamment dans les processus néoplasiques (Barak et al., 2004).

Selon un autre aspect de l'invention, les cellules souches peuvent être différenciées en cellules hématopoiétiques précurseurs et différenciées, caractérisées entre autre par la présence de marqueurs membranaires, connus chez la souris et l'homme par la nomenclature CD (Cluster of Differentiation) et illustrée par Lai et al. dans une revue publiée dans le Journal of Immunology (1998). D'autres marqueurs non membranaires peuvent être également utilisés, notamment des facteurs de transcription spécifiques.

Par « inhibition de l'expression ou de l'activité d'un gène », l'homme du métier comprend que tous les moyens usuels permettant de contrôler l'expression des gènes sont utilisables, notamment l'utilisation d'inhibiteurs de transcription ou de traduction. En particulier l'usage d'ARN interférents permet d'inhiber spécifiquement l'expression de gènes d'intérêt.

Selon un aspect particulier de l'invention, l'inhibition de l'expression ou de l'activité du gène est réalisée de façon conditionnelle. Ainsi la différenciation des cellules peut être déclenchée à un moment souhaité par l'homme du métier.

Selon un aspect particulier de l'invention, l'inhibition de l'expression ou de l'activité du gène est réalisée au moyen d'au moins un ARN interférent.

Les molécules d'ARN interférent sont utilisées pour cibler spécifiquement des ARN messager (ARNm) : l'ARN interférent vient s'hybrider à l'ARNm et par conséquent inhibe la traduction de la protéine correspondante, soit par simple encombrement stérique, soit en favorisant le clivage de l'ARNm.

Cette stratégie d'inhibition de l'expression de gènes a été appliquée aux cellules embryonnaires souches murines à la fois *in vitro* et *in vivo* (Grabarek et al., 2003 ; Kunath et al., 2003 ; Lickert et al., 2005). L'utilisation des RNAi *in ovo* chez le poulet est possible comme cela a été démontré dans (Nakamura et al., 2004).

L'ARN interférent peut être choisi parmi plusieurs formes telles que : ARN antisens, ARN double-brin, « small interfering » ARN, « Small Hairpin» ARN ou ARN ribosomal.

Les siRNA, pour « Small Interfering RNA » ou ARN interférant de petite taille, sont des séquences courtes d'environ 15 à 30 paires de bases (bp), de préférence de 19 à 25 bp. Elles comprennent un premier brin et un brin complémentaire identiques à la région ciblée de l'ARN du gène cible. Par siRNA, on entend les formes d'ARN double brin synthétiques.

Les shRNA pour « Small Hairpin RNA » sont des ARN double brin produits par une séquence clonée dans un vecteur d'expression, codant pour une molécule d'ARN qui adoptera une forme en épingle à cheveux après clivage par le complexe Dicer et loquacious (Du et al ; 2005).

Le design et la préparation d'ARN interférents et leur utilisation pour la transfection de cellules in vivo et in vitro sont bien connues et largement décrites dans de nombreuses publications, telles que: US 6 506 559, US 2003/0056235, WO 99/32619, WO 01/75164, WO 02/44321, US 2002/0086356, WO 00/44895, WO 02/055692, WO 02/055693, WO 03/033700, WO 03/035082, WO 03/035083, WO 03/035868, WO 03/035869, WO 03/035870, WO 03/035876, WO 01/68836, US 2002/0162126, WO 03/020931, WO 03/008573, WO 01/70949, WO 99/49029, US 6573099, WO 2005/00320, WO 2004/035615, WO 2004/019973, WO 2004/015107, http://www.atugen.com/sirnatechnology.htm, http://www.alnylam.com/science-technology/index.asp, http://www.protocol-online.org/prot/Research Tools/Online Tools/SiRNA Design/, http://www.hgmp.mrc.ac.uk/Software/EMBOSS/Apps/sirna.html, http://www.rockefeller.edu/labheads/tuschl/sirna.html, http://www.upstate.com/browse/categories/siRNA.q.

Les siRNA peuvent être conçus et préparés en employant des logiciels appropriés disponibles en ligne, par exemple :
- "siSearch Program" http://sonnhammer.egb.ki.se/siSearch/siSearch 1.6.html ("Improved and automated prediction of effective siRNA", Chalk AM, Wahlesdelt C, and Sonnhammer ELL, Biochemical and Biophysical Research Communications, 2004).
- "SiDirect" http://design.rnai.ip/sidirect/index.php (Direct: highly effective, target-specific siRNA design software for mammalian RNA interference, Yuki Naito et al, Nucleic Acids Res, Vol. 32, No. Web Server issue © Oxford University Press 2004).
- "siRNA design tool" de Whitehead Institute of Biomedical Research au MIT http://jura.wi.mit.edu/pubint/http://iona.wi.mit.edu/siRNAext/
- siRNA wizard^{™} de Invitrogen http://www.sirnawizard.com/,
- "siRNA Target Finder" de Ambion http://www.ambion.com/techlib/misc/siRNA finder.html,
- https://www.genscript.com/ssl-bin/app/mai
- http://www.promega.com/siRNADesigner/default.htm
- http://bioweb.pasteur.fr/seqanal/interfaces/sirna.html
- D'autres programmes sont référencés sur le site http://web.mit.edu/mmcmanus/www/home1.2files/siRNAs.htm, comme http://athena.bioc.uvic.ca/cgi-bin/emboss.pl?_action=input&_app=sirna.

Les outils pour la préparation de siRNA et la transfection de cellules sont à la disposition du public, comme par exemple les vecteurs de siRNA commercialisés par la société Invitrogen (http://www.invitrogen.com ).

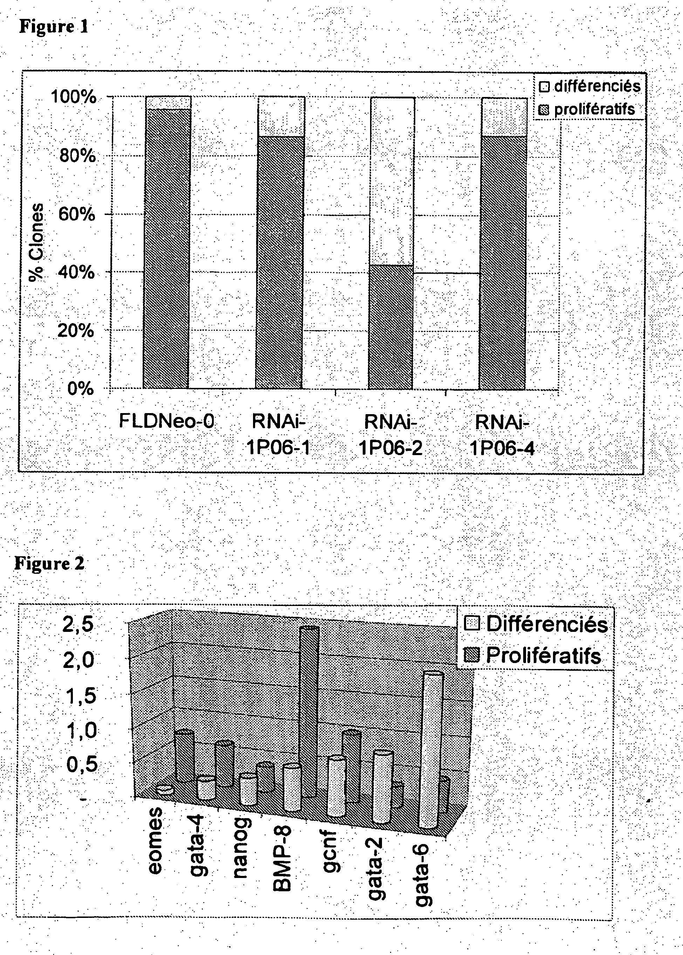

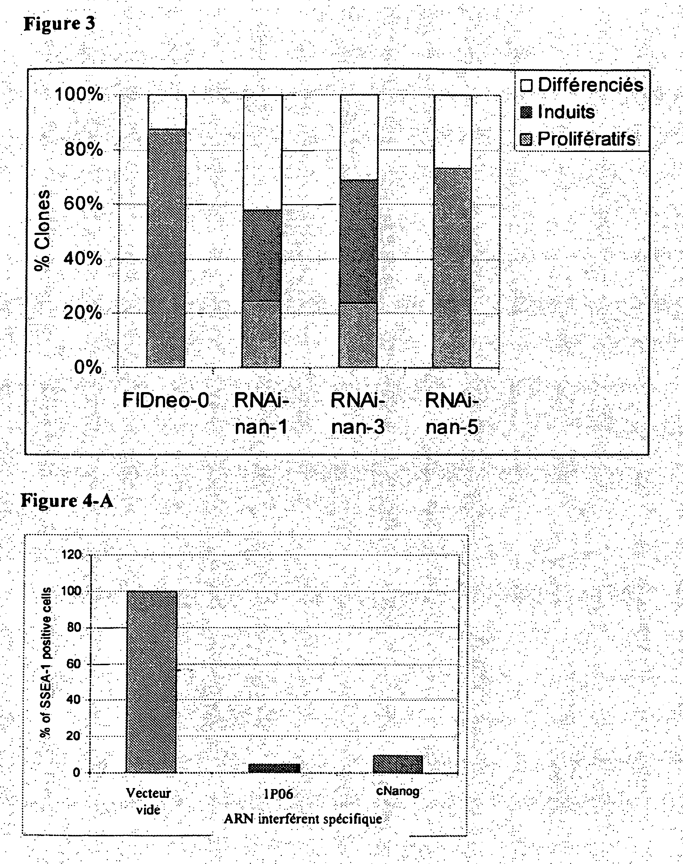

Selon les séquences d'ARN interférents sélectionnées par l'homme du métier, différents niveaux d'inhibition pourront être obtenus, permettant de moduler l'effet inhibiteur recherché. De manière préférentielle, les ARN interférents sont préparés et sélectionnés pour obtenir au moins 50 % d'inhibition de l'expression du gène cible dans une cellule, voire au moins 75%, 90%, 95% voire plus de 99% d'inhibition. L'homme du métier saura sélectionner les ARNi les plus efficaces par simple travail de routine, par exemple en analysant leur capacité à induire la différenciation des cellules souches (voir Figures 1 et 3).

L'ARN interférent inhibe spécifiquement l'expression du gène *Nanog.*

En particulier, la molécule d'acide nucléique codant pour l'ARN interférent peut présenter la séquence SEQ ID N°4 ou SEQ ID N°5.

La molécule avec laquelle la meilleure inhibition d'expression est obtenue est codée par la séquence SEQ ID N° 4.

Une technique courante pour inhiber l'expression d'un gène avec un ARN interférent consiste à introduire dans la cellule un ARN interférent double brin ; mais, comme cet ARNdb sera dégradé rapidement, 1-'inhibition du gène sera limitée dans le temps. Cette limitation a conduit les inventeurs à rechercher un système permettant de produire dans la cellule cible des ARN interférents « à la demande ».

La présente invention concerne des vecteurs d'expression comprenant une molécule d'acide nucléique codant pour un ARN interférent, placée sous le contrôle d'un promoteur permettant l'expression dudit ARN interférent dans une cellule hôte. La cellule hôte sera préférentiellement une cellule souche aviaire maintenue en culture *in vitro.*

Lesdits vecteurs comprennent de préférence un promoteur, des signaux d'initiation et de terminaison de la traduction, ainsi que des régions appropriées de régulation de la transcription.

Selon un mode particulier de l'invention, le promoteur du vecteur d'expression qui contrôle l'expression de l'ARN interférent est un promoteur inductible.

L'avantage d'un système d'expression inductible est que l'on peut maîtriser la durée de présence de l'ARN interférent, et donc de l'inhibition du gène cible. En effet, certains gènes, essentiels à la survie de la cellule, ne peuvent être inhibés trop longtemps car cela induirait la mort cellulaire ; d'autres gènes doivent être inhibés pendant une longue période avant de pouvoir observer les effets de cette inhibition. La maîtrise de l'expression de l'ARN interférent au sein de la cellule hôte permet de contourner ces deux contraintes.

L'homme du métier connaît plusieurs types de promoteurs transcriptionnels inductibles, comme par exemple celui de l'opéron lactose, qui a été le premier identifié et analysé. On peut également citer le système tet off/tet on basé sur un promoteur dérivant de l'opéron de résistance à la tétracycline de E.coli (Gossen et al., 1992).

L'induction de l'activité d'un promoteur peut être réalisée soit en activant un promoteur inactif, soit en levant l'inhibition pesant sur un promoteur actif constitutionnellement. Chaque facteur inhibiteur ou activateur de transcription peut lui-même être inductible en fonction de l'environnement cellulaire.

Par exemple, si le promoteur possède un domaine de fixation à des facteurs inhibiteurs de la transcription, la levée de l'inhibition se fera par le piégeage de ces facteurs qui ne pourront plus se fixer sur les séquences régulatrices du promoteur ; le promoteur sera alors « libéré » des facteurs inhibant la transcription.

Selon un autre exemple, le promoteur est actif constitutionnellement, mais est inhibé par la présence d'une cassette insérée dans son locus ; la cassette est placée de telle façon que les éléments régulateurs du promoteur ne peuvent plus coopérer entre eux car ils sont trop éloignés ; la levée de l'inhibition se fait alors en excisant la cassette, en particulier au moyen de séquences reconnues par une recombinase, placées à chaque extrémité de la cassette. La cassette peut comprendre une longue séquence d'ADN « tampon » servant uniquement à faire de l'encombrement stérique (Tiscomia et al., 2004), mais elle peut aussi comprendre des gènes « marqueurs » comme par exemple des gènes de résistance aux antibiotiques, ou des gènes de fluorescence comme la GFP (Green Fluorescence Protein). La présence de cette cassette de sélection permet également de maintenir le locus dans une configuration chromatinienne active qui limite les mécanismes de 'silencing' des séquences, notamment dans les cellules souches.

Pour obtenir l'excision de la cassette, deux systèmes sont fréquemment utilisés, le système Cre-Lox et le système FLP-FRT. Le système Cre-lox est basé sur l'habilité d'une enzyme, la Cre recombinase, de supprimer tout fragment d'ADN se trouvant entre des séquences précises d'ADN : les séquences loxP. Le système FLP-FRT est lui basé sur l'activité de la FLP recombinase qui supprime les fragments d'ADN se trouvant entre deux séquences FRT. Les sites de reconnaissance Lox P et FRT et les recombinases Cre et FLP correspondantes sont bien connus de l'homme du métier, notamment décrit dans Kilby, et al. ; 1993, Sauer et Henderson, 1988, Gu et al.; 1994, Cohen-Tannoudji et Babinet ; 1998, Shibata et al. ; 1997, Schlake et Bode 1994.

En particulier, un promoteur inductible peut contenir une cassette lox - promoteur de la Thymidine kinase (TK)-Neomycin^{R}-lox dont la présence inhibe son fonctionnement (Coumoul et al., 2004). Le promoteur ne devient fonctionnel qu'après excision par la recombinase CRE de cette cassette. La recombinase utilisée CRE a elle-même une activité dépendante de la présence de tamoxifène dans le milieu de culture (Metzger et al., 1999). Ainsi par ajout de tamoxifène dans le milieu de culture des cellules, la recombinase devient active et excise la séquence inhibitrice du promoteur, rendant ainsi le promoteur fonctionnel, permettant ainsi l'expression de l'ARN interférent.

Parmi les promoteurs d'intérêt, on choisit de préférence les promoteurs pol III dépendants comme le promoteur U6 ou H1, bien connus de l'homme de métier pour leur utilisation dans l'expression des ARN interférents.

Le vecteur d'expression selon l'invention peut être intégré dans une cellule hôte, en particulier une cellule aviaire maintenue en culture *in vitro.* On dit alors que les cellules sont « transformées ». Pour se faire, on peut utiliser des vecteurs à réplication autonome au sein de la cellule hôte, ou des vecteurs intégratifs qui vont permettre l'intégration de la molécule d'acide nucléique exogène dans l'ADN de la cellule hôte.

Parmi les systèmes à réplication autonome, on utilise de préférence des systèmes de type plasmidique ou viral, les vecteurs viraux pouvant notamment être des adénovirus (Perricaudet et al., 1992), des rétrovirus, des lentivirus, des poxvirus ou des virus herpétiques (Epstein et al., 1992). L'homme du métier connaît les technologies utilisables pour chacun de ces systèmes.

Lorsque l'on souhaite l'intégration de la séquence dans les chromosomes de la cellule hôte, on peut utiliser par exemple des systèmes de type plasmidique ou viral; de tels virus sont, par exemple, les rétrovirus (Temin, 1986), ou les AAV (Carter, 1993).

Parmi les vecteurs non viraux, on préfère les polynucléotides nus tels que l'ADN nu ou l'ARN nu selon la technique développée par la société VICAL.

Dans les cellules aviaires, on utilisera préférentiellement des rétrovirus, des adénovirus aviaires, des poxvirus ou bien de l'ADN nu introduit par transfection ou électroporation.

De tels vecteurs sont préparés selon les méthodes couramment utilisées par l'homme du métier, et les clones en résultant peuvent être introduits dans une cellule hôte par des méthodes standard, telles que la lipofection, l'électroporation, le choc thermique, la transformation après perméabilisation chimique de la membrane ou la fusion cellulaire.

Selon un autre aspect de l'invention, le procédé est caractérisé par le fait que les cellules subissent au moins une étape d'activation ou d'inhibition de gènes spécifiques, dans le but d'obtenir une différenciation spécifique desdites cellules.

En particulier, l'utilisation de certains promoteurs et phases codantes permet de contrôler l'expression de gènes qui induisent eux-mêmes la différenciation terminale des cellules dans des voies définies. Ces gènes codent en particulier pour des facteurs de transcription. Parmi ces gènes, on peut citer les régulateurs du lignage myogénique ('MRF') comme *Myf5*, *MyoD*, *Mrf4*, ceux impliqués dans le déterminisme de la lignée adipocytaire comme le gène PPARg, dans le déterminisme neuronal comme *Sox1* et *Nestine,* dans le déterminime de la voie hématopoiétique comme *Gata2* et *Gata3.*

Cette étape d'activation ou d'inhibition de l'expression ou de l'activité de gènes peut être soit simultanée à l'étape d'inhibition du gène *Nanog,* soit décalée dans le temps, et de préférence suivre de quelques heures l'induction de l'inhibition. Ce décalage permet à la cellule de mettre en route le programme de différenciation terminale de façon séquentielle et ainsi de mieux reproduire les cinétiques de différenciation observées *in vivo*.

Selon un autre aspect du procédé de l'invention, les cellules souches aviaires de l'invention sont cultivées dans des conditions appropriées, en particulier en présence de certains facteurs de croissance et inducteurs, pour obtenir une différenciation spécifique desdites cellules.

Parmi ces facteurs, on peut citer les facteurs de croissance de la famille des BMP, des FGF et de la famille wnt dont les rôles relatifs très complexes dans le contrôle des voies mésodermiques et ectodermiques commencent à être décrits à la fois chez la souris, le xénope et le poulet. On utilisera de préférence ces facteurs dans une gamme de concentration de l'ordre de 1 à 10 ng /ml, notamment pour le BMP-4 et le BMP-7. De très nombreux autres facteurs interviennent dans les inductions des voies ecto et mésodermiques.

Le procédé selon l'invention peut également comprendre une étape de sélection et destruction des cellules non-induites en différentiation.

La disparition des clones non induits peut être réalisée de façon concomitante à l'induction, par une sélection négative à l'aide d'un vecteur exprimant un gène toxique (thymidine kinase, toxine diphtérique), placé sous contrôle d'un promoteur actif uniquement dans les cellules souches, par exemple le promoteur du gène *Ens1* ou du gène *1P06* décrit ci-après. Ainsi seules les cellules présentant les caractéristiques de cellules souches disparaîtront.

Un autre objet de l'invention est une molécule d'acide nucléique codant pour un ARN interférent tel que précédemment défini dans l'invention.

L'invention concerne également un vecteur d'expression comprenant une molécule d'acide nucléique codant pour un ARN interférent selon l'invention, placée sous le contrôle d'un promoteur permettant l'expression dudit ARN interférent dans une cellule hôte, de préférence une cellule aviaire maintenue en culture *in vitro*.

Selon un aspect préféré de l'invention, ce promoteur est inductible.

L'invention se rapporte également aux cellules souches aviaires transformées avec un vecteur d'expression tel que décrit ci-dessus.

L'invention concerne également un vecteur de clonage et/ou d'expression dans lequel est inséré une molécule d'acide nucléique présentant l'une des séquences telles que décrites ci-dessus.

L'invention se rapporte également à une séquence d'acide nucléique présentant au moins 90 % d'identité avec la séquence nucléotidique selon la SEQ ID N°10, qui correspond à la région promotrice du gène *Nanog,* c'est-à-dire aux 12112 paires de bases précédant le codon ATG débutant la séquence codante du gène *Nanog* de poulet.

Cette région promotrice du gène *Nanog* contient des éléments de régulation de ce gène, et plus exactement des sites de fixation pour facteurs de régulation transcriptionnelle. Ce gène étant exprimé uniquement dans les cellules souches, l'étude des mécanismes régulant son expression est particulièrement importante pour déterminer les conditions dans lesquelles les cellules souches restent indifférenciées. L'homme du métier saura identifier les facteurs de régulation capables de se fixer sur cette séquence promotrice, ainsi que leurs sites exacts de fixation sur l'ADN, qu'ils soient déjà connus ou qu'ils soient nouvellement identifiés. Pour revue voir (van Steensel B., 2005, Nat Genetics), ( Siggia ED., 2005, Curr Opin Genet Dev) et (Pavesi G, Mauri G, Pesole G, 2004, Brief Bioinform). En particulier des séquences régulatrices déjà connues pourront être identifiées en effectuant des recherches sur les bases de données TRANSFAC, TRRD et COMPEL, qui répertorient des séquences de régulation de l'expression des gènes. Ces bases sont accessibles aux adresses suivantes : http://transfac.gbf.de/TRANSFAC ou http://www.bionet.nsc.ru/TRRD.

Un autre objet de l'invention est un vecteur de clonage et/ou d'expression dans lequel est inséré une molécule d'acide nucléique présentant la séquence telle que définie ci-dessus.

L'invention concerne aussi une cellule procaryote ou eukaryote ayant intégré l'un des vecteurs de clonage et/ou d'expression tels que décrits précédemment.

Les exemples ci-dessous permettent d'illustrer l'invention, et ne doivent pas être considérés comme la limitant.

### DESCRIPTION DES FIGURES

**Figure 3** : L'inhibition de l'expression du gène *Nanog* par des RNAi spécifiques induit la différenciation des cellules CESC et donc un arrêt de la prolifération cellulaire. Cet effet est mesuré sur la morphologie des clones dont la différenciation a été induite par l'hydroxy-tamoxifene.
**Figure 4** : Inhibition de l'expression des gènes *1P06* et *Nanog* par un ARN interférent spécifique :
   A- Marquage des cellules induites en différenciation par l'anticorps anti-SSEA-1
   B- Cinétique de l'arrêt de la prolifération induit par les ARN interférents
   C- Analyse moléculiare de l'expression de gènes marqueurs de différenciation dans les cellules après l'induction de cette différenciation

### EXEMPLES

### EXEMPLE 2: INDUCTION DE LA DIFFERENCIATION DES CELLULES SOUCHES AVIAIRES PAR INHIBITION DE L'EXPRESSION DU GENE NANOG AU MOYEN D'UN ARN INTERFERENT

En réalisant des expériences similaires que celles réalisées sur le gène *1P06,* avec des vecteurs d'expression de RNAi dirigés contre le gène *Nanog,* on observe également une inhibition de la prolifération et une induction de la différenciation des clones sélectionnés. Les clones sont comptés 4 jours (96 heures) après l'induction de la recombinase CRE par l'addition du 4-hydroxytamoxifene. Le nombre total de clones obtenus est respectivement de n = 5 pour le pFLΔNeo-0 (vecteur contrôle vide), n = 21 pour pFLΔNeo-nan-1, n = 37 pour pFLΔNeo-nan-3, n = 15 pour pFLΔNeo-nan-5. Les résultats obtenus sont montrés sur la figure 3.
La figure 3 montre le pourcentage de clones dont la différenciation a été induite (clones induits ou différenciés) et le pourcentage de clones qui sont toujours en prolifération. A l'inverse du vecteur vide, les RNAi-nan1 et RNAi-nan3 sont capables d'induire une différenciation de la majorité des cellules : moins de 30% des clones sont toujours en prolifération. Le RNAi-nan-5 induit également cette différenciation, dans une moindre mesure.

### EXEMPLE 3 : INDUCTION DE LA DIFFERENCIATION PAR INHIBITION DES GENES 1P06 ET NANOG EN PARALLELE

Dans ces expériences, les shRNA utilisés ont été les suivants :
RNAi-2 pour l'inactivation de *1P06,* et RNAi-nan1 pour l'inactivation de *Nanog*

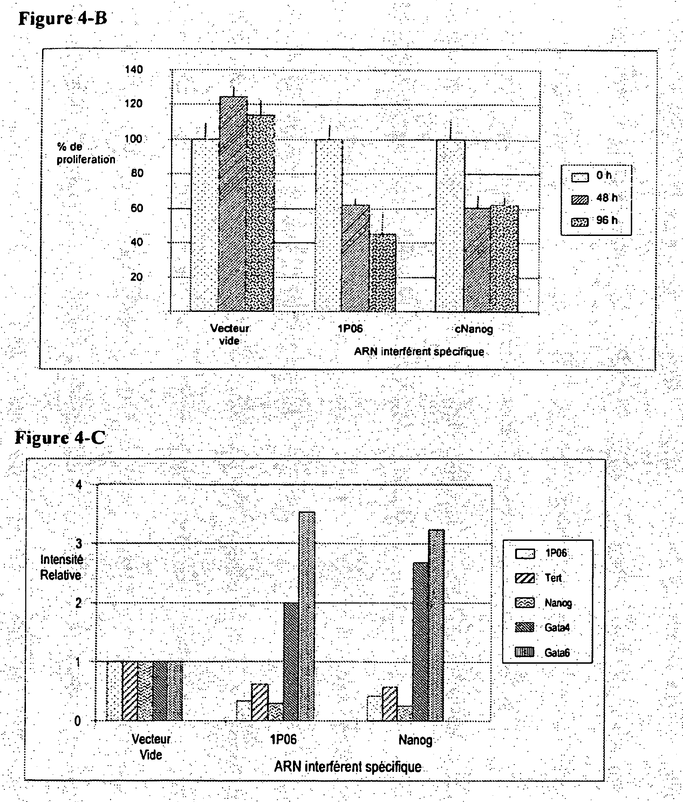

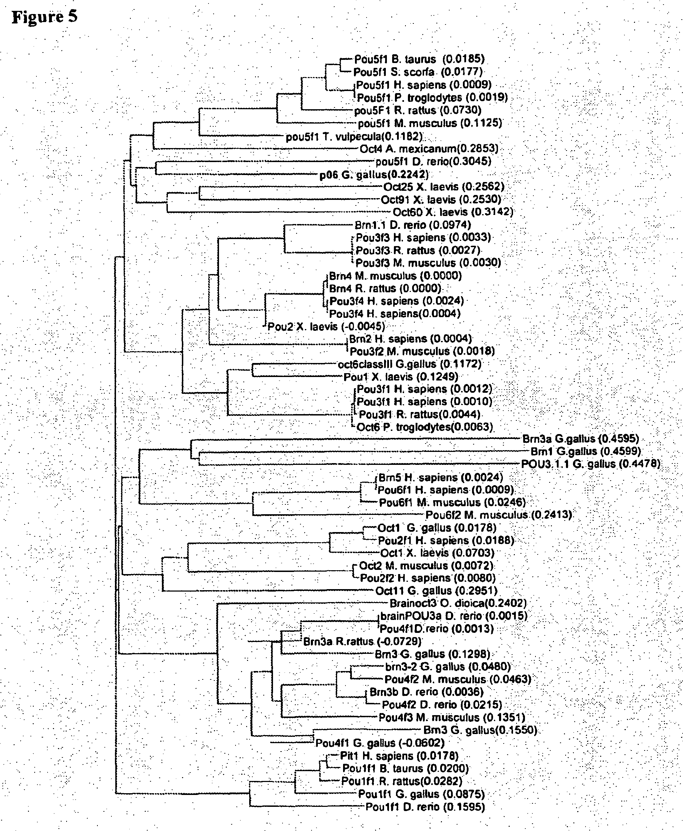

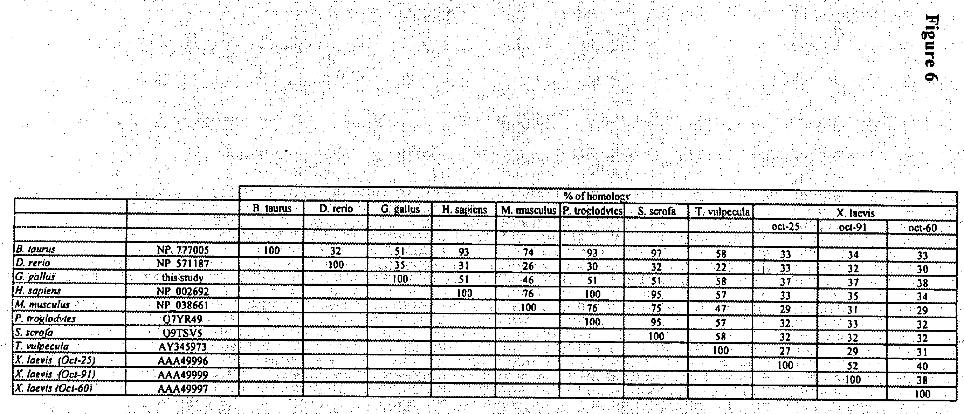

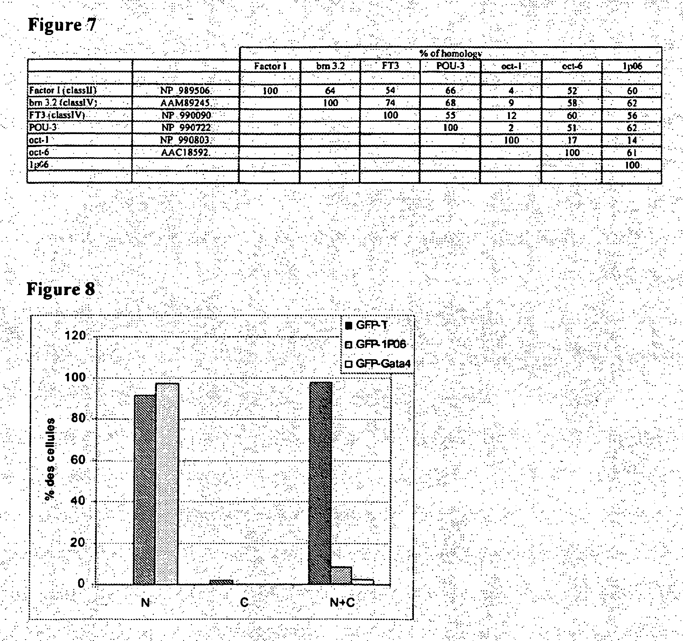

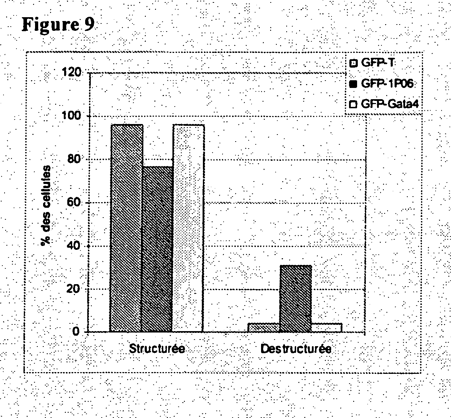

La différenciation des cellules souches aviaires, suite à l'induction par inhibition de l'expression des gènes *1P06* et *Nanog,* a été évaluée par les mesures suivantes :
- pourcentage de cellules positives au marquage par l'anticorps anti-SSEA-1 (Figure 4A)
- pourcentage de prolifération des clones obtenus sur 48 et 96 heures (Figure 4B)
- expression relative de gènes «marqueurs» : *1P06, Tert, Nanog, Gata4, Gata6* (Figure 4C).
La figure 4A montre que les cellules obtenues, en grande majorité, n'expriment plus l'antigène SSEA-1 (Stage-Specific Embryonic Antigen-1), dont l'expression est caractéristique des cellules souches (100% de cellules positives lorsque la transfection est réalisée avec un vecteur vide).
La figure 4B met en evidence la chronologie des événements, et notamment le fait que l'arrêt de la prolifération de clones est obtenu rapidement.
Les clones sont comptés par observation microscopique directe, comme décrit précédemment, à 48 et 96 heures après l'induction par le 4-hydroxy-tamoxifene ; la figure y montre le pourcentage relatif de clones dont la différenciation a été induite ou qui sont toujours en prolifération : après 48h seulement 60% des clones sont encore en prolifération, ceci étant l'effet maximal obtenu en inhibant l'expression du gène *Nanog ;* après 96h d'inhibition du gène *IP06,* on n'observe plus que 40% de clones en prolifération.

La figure 4C montre la caractérisation moléculaire des cellules induites en différenciation par inhibition de l'expression des gènes *IP06* et *Nanog.*
L'expression des différents gènes « marqueurs » est normalisée par rapport au taux d'expression '1' observé dans les clones ayant intégré le vecteur vide pFLDNeo-0.
L'analyse du contenu transcriptomique de ces clones par des approches de RT-PCR en temps réel indique que le niveau global d'expression du gène *1P06* est moins important après différenciation. Les niveaux d'expression des gènes *Tert* et *Nanog* sont diminués. A l'inverse, les gènes *Gata-4* et *Gata-6* ont une expression augmentée, suggérant une induction dans une voie de différenciation équivalente à l'endoderme primitif.

### EXEMPLE 7 : CLONAGE DU PROMOTEUR DU GENE NANOG

Le clonage du promoteur du gène *Nanog* permet de démontrer la présence de sites spécifiques de fixation de facteurs de transcription susceptibles de réguler l'expression de ces gènes dans les cellules souches embryonnaires et germinales aviaires.
Dans les expériences de transfection, les résultats suivants ont été obtenus avec les constructions du promoteur de *Nanog :* ce promoteur est actif exclusivement dans les cellules prolifératives, non différenciées. Ainsi, des transfections transitoires dans les cellules CESC non différenciées et induites en différenciation permettent de montrer que seules les cellules non différenciées sont capables de transactiver ce promoteur. Ainsi des cellules transfectées avec la construction p2000Nanog-GFP montrent une expression de la GFP dans 42% des cellules et seulement dans 7% des cellules quand celles-ci sont traitées pendant 48 h par 10⁻⁷M d'acide rétinoïque.

## Revendications

1. Procédé de préparation de cellules aviaires différenciées à partir de cellules souches aviaires cultivées dans un milieu de culture approprié, **caractérisé en ce qu'**il comprend une étape d'induction de la différenciation des cellules souches par inhibition de l'expression ou de l'activité du gène *nanog* exprimé dans lesdites cellules souches.

2. Procédé selon la revendication 1, **caractérisé en ce que** l'inhibition de l'expression ou de l'activité du gène est réalisée au moyen d'au moins un ARN interférent, de préférence choisi parmi un ARN antisens, un ARN double-brin, un «small interfering » ARN, un « small hairpin » ARN ou un ARN ribosomal.

3. Procédé selon la revendication 2, **caractérisé en ce que** la molécule d'acide nucléique codant pour l'ARN interférent présente la séquence SEQ ID N°4 ou la séquence SEQ ID N°5.

4. Procédé selon l'une des revendications 2 ou 3, **caractérisé en ce que** la molécule d'acide nucléique codant pour l'ARN interférent est intégrée dans un vecteur d'expression, sous le contrôle d'un promoteur permettant l'expression dudit ARN interférent dans une cellule hôte, en particulier sous le contrôle d'un promoteur inductible.

5. Procédé selon la revendication 4, **caractérisé en ce que** le vecteur d'expression comprenant la molécule d'acide nucléique codant pour l'ARN interférent est intégré dans une cellule hôte.

6. Procédé selon l'une des revendications 1 à 5, **caractérisé en ce que** les cellules souches subissent au moins une étape d'activation ou d'inhibition de gènes spécifiques, dans le but d'obtenir une différenciation spécifique desdites cellules.

7. Procédé selon l'une des revendications 1 à 6, **caractérisé en ce que** les cellules souches sont cultivées dans des conditions appropriées pour obtenir une différenciation spécifique desdites cellules.

8. Procédé selon l'une des revendications 1 à 7, **caractérisé en ce qu'**il comprend une étape de sélection et destruction des cellules non-induites en différentiation.

9. Molécule d'acide nucléique codant pour un ARN interférent tel que défini dans la revendication 3, l'ARN interférent présentant la séquence ID n°4 ou la séquence ID n°5.

10. Vecteur d'expression comprenant une molécule d'acide nucléique selon la revendication 9, placée sous le contrôle d'un promoteur permettant l'expression dudit ARN interférent dans une cellule hôte, en particulier sous le contrôle d'un promoteur inductible.

11. Cellules souches aviaires transformées avec un vecteur d'expression selon la revendication 10.

12. Séquence d'acide nucléique codant pour la région promotrice du gène nanog, présentant au moins 90 % d'identité avec la séquence nucléotidique selon la SEQ ID N°10.

13. Vecteur de clonage et/ou d'expression dans lequel est inséré une molécule d'acide nucléique présentant une séquence selon la revendication 12.

14. Cellule procaryote ou eukaryote ayant intégré un vecteur selon la revendication 13.

## Claims

1. Process for preparing differentiated avian cells from avian stem cells cultivated in an appropriate culture medium, **characterized in that** it includes a step of inducing the differentiation of the stem cells by inhibiting the expression or activity of the *Nanog* gene expressed in said stem cells.

2. Process according to claim 1, **characterized in that** the inhibition of the expression or activity of the gene is performed by means of at least one interfering RNA, preferably chosen from an antisense RNA, a double-stranded RNA, a "small interfering" RNA, a "small hairpin" RNA or a ribosomal RNA.

3. Process according to claim 2, **characterized in that** the nucleic acid molecule coding for the interfering RNA has sequence SEQ ID N°4 or sequence SEQ ID N°5.

4. Process according to one of claims 2 or 3, **characterized in that** the nucleic acid coding for the interfering RNA is integrated in an expression vector, under the control of a promoter enabling the expression of said interfering RNA in a host cell, in particular under the control of an inducible promoter.

5. Process according to claim 4, **characterized in that** the expression vector including the nucleic acid molecule coding for the interfering RNA is integrated in a host cell.

6. Process according to one of claims 1 to 5, **characterized in that** the stem cells are subjected to at least one step of activation or inhibition of specific genes, in order to obtain a specific differentiation of said cells.

7. Process according to one of claims 1 to 6, **characterized in that** the stem cells are cultivated under conditions suitable for obtaining a specific differentiation of said cells.

8. Process according to one of claims 1 to 7 **characterized in that** it includes a step of selecting and destroying cells that were not induced into differentiation.

9. Nucleic acid molecule coding for an interfering RNA as defined in claim 3, in which the interfering RNA has sequence SEQ ID N°4 or sequence SEQ ID N°5.

10. Expression vector including a nucleic acid molecule according to claim 9, placed under the control of a promoter enabling the expression of said interfering RNA in a host cell, in particular under the control of an inducible promoter.

11. Avian stem cells transformed with an expression vector according to claim 10.

12. Nucleic acid sequence coding for the promoter region of the *Nanog* gene, having at least 90% identity with the nucleotide sequence according to SEQ ID N°10.

13. Cloning and/or expression vector in which a nucleic acid molecule having a sequence according to claim 12 is inserted.

14. Prokaryotic or eukaryotic cell having integrated a vector according to claim 13.

## Patentansprüche

1. Verfahren zur Herstellung differenzierter Vogelzellen ausgehend von Vogel-Stammzellen, die in einem geeigneten Kulturmedium kultiviert werden, **dadurch gekennzeichnet, dass** es einen Schritt einer Induktion der Differenzierung der Stammzellen durch Hemmung der Expression oder der Aktivität des Gens *nanog,* das in den Stammzellen exprimiert wird, umfasst.

2. Verfahren nach Anspruch 1, **dadurch gekennzeichnet, dass** die Hemmung der Expression oder der Aktivität des Gens mithilfe mindestens einer interferierenden RNA, vorzugsweise ausgewählt aus einer Antisense-RNA (Gegensinn-RNA), einer doppelsträngigen RNA, einer "small interfering" RNA, einer "small hairpin" RNA oder einer ribosomalen RNA, durchgeführt wird.

3. Verfahren nach Anspruch 2, **dadurch gekennzeichnet, dass** das Nukleinsäuremolekül, das die interferierende RNA kodiert, die Sequenz SEQ 10 Nr. 4 oder die Sequenz SEQ ID Nr. 5 aufweist.

4. Verfahren nach einem der Ansprüche 2 oder 3, **dadurch gekennzeichnet, dass** das Nukleinsäuremolekül, das die interferierende RNA kodiert, in einen Expressionsvektor integriert ist unter der Kontrolle eines Promotors, der die Expression der interferierenden RNA in einer Wirtszelle ermöglicht, insbesondere unter der Kontrolle eines induzierbaren Promotors.

5. Verfahren nach Anspruch 4, **dadurch gekennzeichnet, dass** der Expressionsvektor, der das Nukleinsäuremolekül, das die interferierende RNA kodiert, umfasst, in eine Wirtszelle integriert ist.

6. Verfahren nach einem der Ansprüche 1 bis 5, **dadurch gekennzeichnet, dass** die Stammzellen mindestens einen Schritt einer Aktivierung oder einer Hemmung von spezifischen Genen durchlaufen mit dem Ziel, eine spezifische Differenzierung der Zellen zu erhalten.

7. Verfahren nach einem der Ansprüche 1 bis 6, **dadurch gekennzeichnet, dass** die Stammzellen unter Bedingungen kultiviert werden, die geeignet sind, um eine spezifische Differenzierung der Zellen zu erhalten.

8. Verfahren nach einem der Ansprüche 1 bis 7, **dadurch gekennzeichnet, dass** es einen Schritt eines Selektierens und eines Zerstörens der nicht zur Differenzierung induzierten Zellen umfasst.

9. Nukleinsäuremolekül, welches eine interferierende RNA, wie in Anspruch 3 definiert, kodiert, wobei die interferierende RNA die Sequenz ID Nr. 4 oder die Sequenz ID Nr. 5 aufweist.

10. Expressionsvektor, umfassend ein Nukleinsäuremolekül nach Anspruch 9, das unter die Kontrolle eines Promotors, der die Expression der interferierenden RNA in einer Wirtszelle ermöglicht, insbesondere unter die Kontrolle eines induzierbaren Promotors gestellt ist.

11. Vogel-Stammzellen, die mit einem Expressionsvektor nach Anspruch 10 transformiert sind.

12. Nukleinsäuresequenz, welche die Promotorregion des Gens *nanog* kodiert, welche mindestens 90% Identität mit der Nukleotidsequenz gemäß SEQ ID Nr. 10 aufweist.

13. Klonierungs- und/oder Expressionsvektor, in welchen ein Nukleinsäuremolekül, welches eine Sequenz gemäß Anspruch 12 aufweist, inseriert ist.

14. Prokaryotische oder eukaryotische Zelle, welche einen Vektor nach Anspruch 13 integriert hat.
